# EUROPEAN PATENT APPLICATION

(11) **EP 1 681 052 A1**
(43) Date of publication of application: **19.07.2006**
(21) Application number: 06250256.2
(22) Date of filing: 18.01.2006
(51) Int. Cl.: A61K 9/32, A61K 9/36, A61K 9/38, A61K 9/58, A61K 9/62, A61K 9/64, A61K 31/4439, A61K 9/48

(54) **Solid pharmaceutical dosage forms for reducing the bioavailability food effect**

(30) Priority: 18.01.2005 US 37462
(71) Applicant: Dr. Reddy's Laboratories Ltd., Hyderabad 500 016 (IN); Dr. Reddy's Laboratories, Inc., Bridgewater, NJ 08807 (US)
(72) Inventor: Mohan, Mailatur Sivaraman c/o No.508 5th Floor, Kukutapally Post Office, Hyderabad (IN); Bhushan, Indu, Hyderabad (IN); Pergament, Edward D., East Brunswick, New Jersey 08816 (US); Bhagwatwar, Harshal Prabhakar c/o Plot 5 9, Hyderabad (IN)
(74) Representative: Bates, Philip Ian

(57) **Abstract**

A method of treatment to avoid bioavailability food effects and improve bioavailability variability, by administering a pharmaceutical dosage form containing a pharmaceutical active agent and a disintegrant in a core, a swellable coating surrounding the core, and an optional enteric coating surrounding the swellable coating.

## Description

### CROSS-REFERENCE TO RELATED APPLICATIONS

This application is a continuation-in-part of copending United States Patent Application 10/893,563 filed on July 16, 2004, which claims the benefit of United States Provisional Application No. 60/563,707 filed April 20, 2004, and which claims priority from the following patent applications that were filed in India: Application No. 580/CHE/2003 filed July 17, 2003; and Application No. 1064/CHE/2003 filed December 30, 2003. This application also claims the benefit of United States Provisional Application No. 60/620,256 filed October 19, 2004. The entire content of each of these prior applications is hereby incorporated by this reference.

### INTRODUCTION TO THE INVENTION

Throughout this application, several patent and other documents are mentioned. The contents of these documents are hereby incorporated by reference.

The invention relates to reducing the bioavailability food effect of solid pharmaceutical dosage forms, by administering dosage forms having exterior coatings that protect contained pharmaceutical active ingredients against degradation by acidic gastric fluid. In particular, the dosage forms have inner coatings comprising substances that swell upon contact with aqueous fluids.

A number of pharmaceutical active ingredients are not chemically stable in acidic environments. For this reason, oral administration cannot be effective without some means for protecting the substances against contact with gastric fluid. This, however, also has the generally undesired effect of delaying availability of the substance to the body, since systemic absorption will not commence until the substance has been released from its dosage form.

Approaches have been devised to protect pharmaceutical dosage forms from being affected by the acidic stomach contents, and permitting active ingredients to be made available only after the dosage form enters a more alkaline environment, such as in the duodenum, jejunum, or ileum. This typically involves coating the dosage form or particles containing an active pharmaceutical agent with a material that resists acid attack, but dissolves or becomes permeable in a more alkaline environment.

Lovgren et al., in U.S. Patent 4,786,505, describe a stable pharmaceutical preparation of omeprazole that resists acid attack, but dissolves rapidly in neutral or alkaline media. Particles of omeprazole are mixed with a water-soluble alkaline-reacting substance and the particles are coated with a "separating layer" that acts as a pH buffering zone to prevent contact of the drug and acidic groups that are present in the final coating material. Finally, the bi-layered composition is coated with an enteric polymer coating that does not react with acids.

U.S. Patent 5,035,899 to Saeki et al. relates to compositions of acid-unstable drugs, which are protected against contact with gastric acid. A core that contains the drug is coated first with fine particles of a material having a low water solubility, then are coated with an enteric film-forming material such as ethylcellulose.

Mazer et al., in U.S. Patent 5,160,742, discloses a sustained release system for an acid-sensitive drug such as a β-lactam antibiotic. Coated drug particles, suitable for inclusion in syrups or other formulations are prepared by forming a core that contains the drug, coating the core with a prolamine, and applying a final exterior coating of an enteric substance such as a methacrylic acid copolymer. Optionally, an additional coating of prolamine can be applied onto the enteric coating layer. The drug is released over a prolonged time, beginning after the coated particles enter a high-pH environment.

U.S. Patent 5,472,712 to Oshlack et al. teaches controlled release formulations having drug-containing core and a controlled release hydrophobic coating of ethylcellulose, optionally containing a hydrophilic pore forming substance such as hydroxypropyl methylcellulose. Optionally, the cores can have an intermediate "barrier" coating of a substance such as hydroxypropyl methylcellulose, which preferably does not affect the dissolution rate of the final product.

In U.S. Patent 5,609,909 to Meyer et al., oral formulations in which the unpleasant taste of a drug substance is masked, but in which the drug is immediately bioavailable upon exposure to acidic fluid in the stomach, are prepared by coating a drug-containing core particle with a mixture of a prolamine and a nonpolymeric plasticizer.

U.S. Patent 5,811,388 to Friend et al. teaches the preparation of a dosage form in which drug is not released to the upper gastrointestinal tract, but is released in the lower gastrointestinal tract for directly treating diseases of the colon. The dosage form includes a core tablet containing the drug and a large amount of a plant-derived hydrocolloid, optionally coated with a film of an enteric substance.

Lerner et al. describe, in U.S. Patent 5,840,332, a composition that delivers a drug to a particular portion of the gastrointestinal tract, wherein a drug-containing core is coated with a water-insoluble material having embedded particles of water-insoluble hydrophilic matter. The coated core can optionally be further coated with an enteric polymer.

U.S. Patent 6,346,269 to Hsiao et al. teaches oral formulations for acid-sensitive drugs, where the drug substance is mixed with an alkaline material such as trisodium phosphate and coated onto a core, such as a tablet, then an enteric coating is applied over the drug substance layer.

Methods for the production of films, sheets, and articles from zein are taught in U.S. Patent 6,635,206 to Padua et al.

A further problem exists with many drug substances, in that the pharmacokinetic properties of the drug are affected by the presence or absence of food in the stomach when a dose is administered, or before the drug has passed from the stomach. This "food effect" has been observed with a diversity of drugs, some being formulated in a controlled or delayed release composition, others being formulated in an immediate release composition. For example, according to their prescribing information, all of the benzimidazole proton pump inhibitor products are affected by the presence of food in the stomach at the time of dosing or shortly thereafter: omeprazole in a 40 mg delayed release capsule does not show a food effect with applesauce, but the 20 mg delayed release capsule gives a 25% reduction in Cₘₐₓ when administered with applesauce; delayed release capsules of esomeprazole magnesium exhibit a decrease of the drug AUC amounting to 43-53% when administration occurs after food intake; the rabeprazole sodium product gives a delay in Tₘₐₓ of 4 hours or longer when administered with a high fat meal; the pantoprazole sodium delayed release product has a highly variable Tₘₐₓ, which can increase significantly when given with food; and delayed release formulations of lansoprazole have both Cₘₐₓ and AUC diminished by about 50-70% if the drug is given 30 minutes after food.

For the commercial product NEXIUM™ esomeprazole magnesium trihydrate, sold by AstraZeneca LP, the New Drug Application (number 21-153) that was submitted to the U.S. Food and Drug Administration has information relating to three food effect bioavailability studies that were conducted. The following table summarizes certain results reported from three studies with single esomeprazole doses, as stated at the U.S. Food and Drug Administration website http://www.fda.gov/cder/foi/nda/2001/21154_Nexium_biopharmr_P1.pdf:

| | **QBE-0025** | | **QBE-0030** | | **QBE-0044** | |
|---|---|---|---|---|---|---|
| | **Fast** | **Fed** | **Fast** | **Fed** | **Fast** | **Fed** |
| Cmax | 3.70 | 1.17 | 2.44 | 1.07 | 2.81 | 0.59 |
| AUC | 6.96 | 3.92 | 4.07 | 2.73 | 4.01 | 1.87 |

N. A. Kshirsagar et al. reported in "Effect of Food on Doxycycline Absorption," *Joumal of Postgraduate Medicine,* Vol. 33, pages 117-119 (1987) that a standard breakfast reduced the bioavailability of doxycycline as judged by AUC and Cₘₐₓ; the drug was given in the form of capsules, and it does not appear that the dosage forms were other than immediate release. Patrick Smith, "Effect of Food on Antiretroviral Pharmacokinetics," posted at the website http://hiv.buffalo.edu/foodeffectson.shtml and dated November 12, 2001 has a listing of drugs that have their bioavailability affected by the presence of food, including: amprenavir, indinavir, nelfinavir, ritonavir, saquinavir, didanosine, lamivudine, zalcitabine, zidovudine, and efavirenz; in some instances, the bioavailability is enhanced by food, while in others the bioavailability is reduced.

A need exists for treatments with a drug-containing dosage form in which drug substances exhibit a predictable bioavailability, whether or not the dosage form is administered with food. Also needed is a treatment method that minimizes inter-patient differences in drug bioavailability parameters.

### SUMMARY OF THE INVENTION

In one embodiment, the invention includes a method of treatment with a pharmaceutical dosage form comprising: a solid core comprising a pharmaceutical active and a disintegrant; a swellable coating surrounding the core; and optionally an enteric coating surrounding the swellable coating. The dosage form can have different embodiments, including coated tablets or capsules containing coated pellets or coated minitablets.

One aspect of the invention involves a dosage form in which a pharmaceutical active is substantially retained while the dosage form is present in the stomach, but where the pharmaceutical active is rapidly released after the dosage form enters an environment having a pH value at least about 5.

Also included in the invention is treatment with a pharmaceutical dosage form comprising: a solid core comprising an acid-sensitive pharmaceutical active and a disintegrant; a swellable coating comprising a hydrocolloid-forming component, surrounding the core; and an enteric coating surrounding the swellable coating.

The invention further includes treatments with a pharmaceutical dosage form comprising: a solid core comprising a benzimidazole and a disintegrant; a swellable coating comprising one or more hydrocolloid-formers selected from zein, crospovidone, and a hydroxypropyl cellulose, surrounding the core: and an enteric coating comprising a copolymer of methacrylic acid and ethyl acrylate, surrounding the swellable coating.

Another aspect of the invention is a method of treating a medical condition comprising orally administering a pharmaceutical dosage form according to any of the preceding aspects and embodiments, in which method: the dosage form remains substantially intact during stomach transit; the enteric coating is removed in digestive system environments having pH values above about 5; aqueous fluids penetrate areas of the dosage form where the enteric coating has been removed, causing hydrocolloid formation in the swellable coating; aqueous fluids pass through the hydrocolloid to hydrate the core; and the hydrated core becomes fragmented, releasing the pharmaceutical active from the dosage form.

A still further aspect of the invention is a method of treating a medical condition comprising orally administering a pharmaceutical dosage form, according to a preceding aspect, that remains substantially intact during stomach transit, then permits aqueous fluids to pass through a swellable coating to hydrate the core; allowing the hydrated core to become fragmented and thereby releasing the pharmaceutical active from the dosage form.

Preferred swelling agents in the swellable coating include prolamines; vinylpyrrolidone polymers; cellulose derivatives; starches; carboxyvinyl polymers; alginates; pectins; agar; and gums. Zein, crospovidone, or a hydroxypropyl cellulose are more preferred for use as the swelling agent.

### DETAILED DESCRIPTION

The subject invention includes a method of treatment that provides a predictable bioavailability of drug substances that ordinarily have a "food effect," i.e., showing a bioavailability difference between administering the drug in a fasted and a fed state.

The term "equivalent" indicates a fed state bioavailability between about 80 percent and 125 percent of the bioavailability from administration in a fasted state, with a 90 percent confidence interval. "Bioavailability" includes both the "Cₘₐₓ" value (maximum plasma concentration of the drug after administration, occurring at an elapsed time called "Tₘₐₓ"), and the "AUC" value (area under the plasma concentration-time curve). Guidelines for appropriate studies have been promulgated by the U.S. Food and Drug Administration, in a December 2002 publication entitled "Guidance for Industry - Food-Effect Bioavailability and Fed Bioequivalence Studies."

The method involves administering a pharmaceutical dosage form comprising a core that comprises a pharmaceutical active ingredient, and a swellable coating surrounding the core. The core comprises at least 50%, at least 60%, at least 70%, at least 80%, at least 82.5%, at least 85%, at least 87%, at least 88%, or at least 89% of the total pharmaceutical composition. The core may also comprise at least 90%, at least 91%, at least 92% or at least 93% of the total pharmaceutical composition.

In this application, the terms "pharmaceutical active ingredient" "pharmaceutical active" and "active" are used interchangeably to refer to a component of a pharmaceutical dosage form that provides a therapeutic effect upon administration to a subject. This invention is particularly applicable to acid-sensitive pharmaceutical actives, which exhibit instability in a low-pH environment, such as the benzimidazole derivatives, including their optically active isomers. Specific examples of useful benzimidazole compounds include rabeprazole, omeprazole, esomeprazole, lansoprazole, and pantoprazole. Other drugs for which the invention will be useful include, without limitation thereto:
pharmaceutical actives that react with enteric coating components, examples being drugs that form insoluble complexes with the enteric coatings, such as fluoxetine and duloxetine; and highly alkaline drugs that can react with acidic groups to reduce the acid-insolubility of the coating, such as diclofenac sodium and piroxicam.

Moreover, in addition to acid-sensitive drugs, the invention is useful for drugs that do not exhibit adverse reactions with stomach acid, enteric coatings, or other acidic substances, but which exhibit a "food effect." The food effect can result in either positive or negative bioavailability changes, and causes the bioavailability to be unpredictable. Patients cannot always precisely follow prescription directions to "take one hour before a meal," or "take two hours after a meal." Providing dosage forms that can be administered without regard to a patient's meal schedule, or even in the absence of a definite meal schedule, is a significant step toward reducing the variability of treatment efficacy.

Food can alter the bioavailability of a drug by different mechanisms, including: delayed gastric emptying; stimulation of bile flow; changed gastrointestinal pH; increased visceral blood flow; changed luminal metabolism; and physical or chemical interactions of food components with the drug compound or dosage form.

A further aspect of the invention is a method of treatment that decreases the variability in bioavailability parameters that is frequently observed between patients. This variability causes uncertainty in establishing dosage amounts and frequencies, and can be particularly problematic when the drug substance being administered has a narrow therapeutic window (i.e., the difference between a therapeutic plasma concentration of the drug and a toxic plasma concentration is not large). By establishing a particular environment for a predictable drug release from the dosage form, the inter-patient variability is minimized.

As contemplated herein, a "swellable coating" is a coating that increases in volume upon contact with aqueous fluids. This swelling usually occurs through imbibition of water. The swellable coating adds 0.1-10%, 0.5-8%, 0.7-7%, 1-5%, 1.3-3%, 1.5-2%, about 2%, or about 1.5% to the weight of the core. In another embodiment, the swellable coating adds 0.1- 5%, 0.1- 4%, 0.1- 3%, 0.1-2%, or 0.1-1% to the weight of the core.

Generally, the swellable coating, upon wetting, becomes a hydrocolloid, which is a gelatinous suspension of microscopic particles in water. Preferably, the hydrocolloid is formed from a prolamine, such as gliadan, hordein, or, more preferably, zein. Zein is extracted from corn as a granular, straw to pale yellow colored amorphous powder or fine flakes and various commercial extracts have molecular weights in the range of 25,000-35,000. Zein is insoluble in water and insoluble in alcohols, but soluble in aqueous alcohol solutions. Chemically, zein is fairly abundant in glutamine and devoid of lysine and tryptophan. Zein comprises about 20-22% glutamic acid and glutamine, 17-20% leucine, 5-9% proline, 8-10% alanine, 4-7% phenylalanine, 3-7% isoleucine, 4-6% serine, 4-5% asparagine and 3-5% tyrosine. All of the other amino acids in zein each comprise less than 3%. Zein has been generally recognized as safe (GRAS) by the United States Food and Drug Administration since March, 1985 for use in food and pharmaceutical products. Zein is available commercially from several sources, including Freeman Industries LLC, Tuckahoe, New York USA; among the commercial zein products sold by this company are those designated Zein F4000, Zein 4400, Zein F6000, Zein G-10, Aqua Zein, and Aqua Zein Neutral.

A presently preferred zein for the present invention is the Zein F6000, which has been re-extracted to reduce its color (from xanthophyll) level. Zein F6000 is a very light yellow granular powder with an approximate molecular weight of 35,000 and a bulk density of 0.125-0.21 g/ml. It contains 90-96% zein protein, calculated on a dry basis.

The hydrocolloid can also be formed from a hydroxypropyl methylcellulose. The viscosity of a 2 weight percent aqueous solution of various hydroxypropyl methylcellulose products ranges from about 4,000 mPa·s to about 100,000 mPa·s. In one embodiment, the hydroxypropyl methylcellulose is United States Pharmacopeia Substitution Type 2208, also called hypromellose 2208, with a viscosity of about 15,000 mPa·s, which is commercially available as Methocel K15M. In another embodiment, the hydroxypropyl methylcellulose is United States Pharmacopeia Substitution Type 2910, also known as hypermellose 2910, with a viscosity of about 4,000 mPa·s, which is marketed as Methocel E4M. METHOCEL is a trademark of Dow Chemical Company, Midland, Michigan U.S.A.

Other useful substances for forming a hydrocolloid include, without limitation, crospovidone; croscarmellose sodium; cellulose derivatives such as hydroxyethylcellulose, hydroxypropyl cellulose, or methylcellulose; gums such as seaweed extracts, plant extracts, plant exudates, plant seed extracts, and microbial fermentation products; starches including pregelatinized and modified starches; and synthetics such as carboxyvinyl polymers, including carbopols. Additional specific examples include alginates, pectins, low methoxy pectins, agar, carrageenan, plus arabic, tragacanth, karaya, ghatti, locust bean (carob), guar, dextran, xanthan, carrageenan, tara, Khaya grandfolia, gellan, Konjac mannan, galactomannan, funoran, acetan, welan, rhamsan, furcelleran, succinoglycan, scieroglycan, schizophylan, curdlan, pullulan, karaya and tamarind gums.

In addition to the pharmaceutical active, the core further comprises a disintegrant that, in an aqueous environment, assists in the physical fragmentation of any material with which is it combined. A disintegrant does not promote dissolution or a chemical change in the material being fragmented. The following are examples of useful disintegrants: starches such as potato or tapioca starch, modified starches (such as sodium starch glycolate) and partially pregelatinized starches (such as Starch 1500); polyvinylpyrrolidones, including modified polyvinylpyrrolidones (such as crospovidone, polymerized under conditions that promote crosslinking); celluloses such as microcrystalline cellulose, modified celluloses (such as low substituted hydroxypropyl cellulose, croscarmellose sodium and calcium carboxymethyl cellulose); formaldehyde-casein compounds (such as Esma-Spreng.RTM); resins, such as the polacrilin potassium sold by Rohm and Haas Company, Philadelphia, Pennsylvania U.S.A., using the trademark AMBERLITE IRP88; defatted soybean extracts; alginic acid; agar-agar; calcium carbonate; calcium phosphate; and sodium carbonate. U.S. Patent 6,696,085 to Rault et al. teaches that acrylic polymers are useful as tablet disintegrants.

In addition to the foregoing, the core can contain any desired components such as binders, lubricants, antioxidants, etc., as are well known in the art and further discussed below.

The pharmaceutical dosage form, in some embodiments, further comprises an enteric coating surrounding the swellable coating. An "enteric coating" is a coating that is substantially insoluble at the acidic pH conditions of the stomach but is substantially soluble or water-permeable at the higher pH conditions of the intestines. In this invention, the enteric coating protects the swellable coating against contact with the acidic stomach environment but permits contact of the swellable coating with the more alkaline intestinal fluid. The enteric coating can be chosen to provide targeted release to a particular section of the intestine. For instance, an enteric coating can provide delivery to the duodenum (pH > 5.5), to the jejunum (pH 6-7), or to the ileum (pH up to 7.5). Intermediate delivery points can be achieved by combining different coating materials or varying the thickness of the coating. Enteric coating materials include cellulose-based coatings, such as cellulose acetate phthalate and hydroxypropylmethyl cellulose phthalate, methacrylate-based coatings, polyvinyl acetate phthalate-based coatings, and shellac-based coatings.

In the present invention, methacrylate-based coatings are preferred and several useful products are commercially available from Röhm GmbH & Co., Darmstadt, Germany under the trademark EUDRAGIT. EUDRAGIT L100-55 is especially preferred. EUDRAGIT L 100-55 is a powder, spray-dried EUDRAGIT L 30 D-55 which can be reconstituted. EUDRAGIT L 30 D-55 is an aqueous dispersion of a pH dependent polymer soluble at or above pH 5.5 for targeted delivery in the duodenum. EUDRAGIT L 100-55 retains the pH dependency of EUDRAGIT L 30 D-55 and thus, is soluble at or above pH 5.5 and provides delivery to the duodenum. EUDRAGIT L 100-55 and EUDRAGIT L 30 D-55 are copolymers of methacrylic acid and ethyl acrylate in a 1:1 ratio. They have the molecular formula: (C₅H₂O₂·C₄H₆O₂)ₓ and have been assigned the Chemical Abstracts Registry No. 25212-88-8. EUDRAGIT L100-55 also meets the United States Pharmacopeia specificiation for Methacrylic Acid Copolymer Type C.

In one embodiment, the enteric coating comprises 1-40%, 3-35%, 5-30%, 6-20%, or 7-10% or 8% of the total composition. In another embodiment, the enteric coating comprises at most 20%, at most 17.5%, at most 15%, at most 12.5%, at most 10%, at most 9%, at most 8%, at most 7%, at most 6%, at most 5%, or at most 4% of the total composition. However, depending on the acid-sensitivity of the pharmaceutical active and/or the water-permeability of the swellable coating, some formulations will not need to have an enteric coating.

When an enteric coating is not used, then it will be important to increase the thickness and/or decrease the water permeability of the swellable coating, with the objective of keeping the dosage form substantially intact during its passage through the stomach. Such dosage forms will be particularly useful for drug substances that can be absorbed throughout the digestive tract, but which exhibit a food effect; use of the compositions described herein will typically prolong the initial Tₘₐₓ due to maintaining the physical stability of the dosage form until a higher pH environment is reached, but the plasma concentration profile of the pharmaceutical active will be more predictable after the dose is administered. This predictability results from releasing the pharmaceutical active into the more environmentally consistent high-pH digestive tract areas. In any event, after administering a few doses, a "steady state" condition of plasma concentrations will typically be attained and the initial delay in Tₘₐₓ will not significantly affect the therapeutic method.

Optionally, an excipient that modulates the release of the pharmaceutical active is added to the swellable coating. Modulation may be achieved by facilitating or impeding the access of water to the core. Useful excipients include plasticizers such as lactic acid, lactic acid acetamide, glycerin, glyceryl monostearate, triacetin, sorbitol, triethyl citrate, polyvinylpyrrolidone, triethylene glycol, tricresyl phosphate, dibutyl tartrate, ethylene glycol monooleate, palmitic acid, stearic acid, oleic acid, dibutyl sebacate, acetylated monoglycerides, and other oils and waxes, as well as polyethylene glycol 300, 400, 600, 1450, 3350 and 8000. Additional excipients that modulate the rate of release of the active include water soluble surfactants, such as sodium lauryl sulfate and docusate sodium, and enteric coating materials, such as EUDRAGIT L 100-55, which are mixed into the swellable coating.

Without being limited to any single theory of operation, it is believed that an enteric coating material that is incorporated into the swellable coating dissolves upon contact with the intestinal fluid and forms channels in the swellable coating, which facilitate the entry of the intestinal fluids into the core. In one embodiment, the enteric coating material constitutes about 0.1-30%, 0.5-20%, 1-17.5%, preferably 5-15%, or more preferably 5-10% of the swellable coating. In another embodiment, the enteric coating material comprises 10-50%, 15-40%, preferably 20-30% of the swellable coating.

Again, without being limited to any theory, it is postulated that the water soluble surfactant causes rapid wetting of the swellable coating upon exposure to the intestinal fluids, thereby assisting entry of fluid into the core. When the water soluble surfactant is present, it constitutes about 0.001-30%, 0.005-20%, 0.01-10%, 0.03-8%, 0.05-6%, 0.07-4%, 0.09-2%, or 0.1-1% by weight of the swellable coating. A preferred range is 0.01-10%.

For instance, where zein is present in the swellable coating, the extent of the swelling controls its permeability and the greatest permeation is achieved at the largest swelling volume. See Y. K. Oh et al., "Swelling and Permeability Characteristics of Zein Membranes," *PDA Journal of Pharmaceutical Science and Technology,* Vol. 57, pages 208-217 (2003) for additional information concerning diffusion through hydrated zein films.

The addition of plasticizers to zein affects its permeability to water. The combination of zein with hygroscopic plasticizers such as glycerol, triethyelene glycol, and levulinic acid produces more water absorption than in unplasticized zein. However, incorporating into zein hydrophobic plasticizers such as dibutyl tartrate and oleic acid results in less water absorption than unplasticized zein. The greater the degree of water permeation, the weaker the tensile strength and the coating can simply give way to provide full release of the pharmaceutical active. See J. W. Lawton, "Plasticizers for Zein: Their Effect on Tensile Properties and Water Absorption of Zein Films," *Cereal Chemistry,* Vol. 81, pages 1-5 (2004) for a discussion of the water absorption characteristics of cast plasticized zein films.

The modulation of the release profile of the pharmaceutical active by an excipient, such as a plasticizer, is not limited to zein. In general, varying the amount and type of plasticizer affects the tensile strength of coatings. The use of hygroscopic versus hydrophobic excipients also affects the release profile in the same manner as discussed regarding zein.

To form the cores of the invention, the pharmaceutical active is blended with one or more pharmaceutically acceptable carriers, such as water, saline, sodium citrate or dicalcium phosphate, and/or any of the following: fillers or extenders, such as starch, lactose, sucrose, glucose, mannitol, or silicic acid; binders, such as carboxymethylcellulose, alginates, gelatin, copolyvidonum (such as the PLASDONE™ S-630 copolymer of N-vinyl-2-pyrrolidone and vinyl acetate, sold by International Specialty Products, Wayne, New Jersey U.S.A,), copolymers of ethylene oxide and propylene oxide such as Poloxamer 407, sucrose, or acacia; humectants, such as glycerol; disintegrants, such as starch, polyvinyl pyrrolidones, celluloses, formaldehyde-casein compounds, defatted soybean extracts, alginic acid, agar-agar, calcium carbonate, calcium phosphate, potato or tapioca starch or sodium carbonate; lubricants such as talc, calcium stearate, magnesium stearate or solid polyethylene glycol; solution retarding agents, such as paraffin; absorption accelerators, such as quaternary ammonium compounds; wetting agents, such as cetyl alcohol and glycerol monostearate; surfactants, such as sodium lauryl sulfate or docusate sodium; absorbents, such as kaolin or bentonite clay; and stabilizing agents. The pharmaceutical active may also be blended with buffering agents such as alkali metal carbonates and alkaline earth metal oxides. This listing is not exhaustive, many other functional components that are known in the art will also be useful in the present invention.

The cores of the invention can be in the form of tablets, minitablets, granules, particulates or pellets. The tablets and minitablets can be manufactured by direct compression or any other process known to those of skill in the art. Dry granulation, wet granulation, melt granulation, or any other process known to those of skill in the art may be used to form granules. The particulates and pellets may be manufactured by any method known to those of skill in the art, such as extrusion or spheronization. Pellets may also be made by melt pelletization or by coating non-pareil seeds. Wet cores are dried by conventional drying procedures such as air drying, or drying under heated and/or low pressure conditions.

The cores of invention are coated with a swellable coating, followed by the optional application of an outer enteric coating. In general, coatings may be applied by any techniques known in the art, such as pan coating (including perforated closed system pan coating), coacervation, or fluidized bed coating. The fluidized bed may contain a rotor insert and/or a Wurster column insert. The coatings can be generally classified according to their polymer base, such as: cellulose-based, including cellulose acetate phthalate, hydroxypropylmethyl cellulose phthalate, hydroxypropylmethyl cellulose, hydroxypropyl cellulose, hydroxypropylethyl cellulose, ethyl cellulose, methyl cellulose, microcrystalline cellulose; carrageenan; methacrylate- or methacrylic acid-based, such as methacrylic acid, methacrylate, acrylate, methacrylate, ethacrylate, methylmethacrylate, or copolymers thereof; or polyvinyl acetate phthalate-based. Typically, the polymer is combined with a solvent, such as water, and a plasticizer, such as polyethylene glycol, lactic acid, lactic acid, acetamide, glycerin, glyceryl monostearate, triacetin, sorbitol, triethyl citrate, polyvinylpyrrolidone, triethylene glycol, tricresyl phosphate, dibutyl tartrate, ethylene glycol monooleate, palmitic acid, stearic acid, oleic acid, or dibutyl sebacate. Optionally, one may also add any of the following elements: an anti-tack agent, an anti-foam agent, a filler, a surfactant, a colorant, a flavoring agent, and combinations of any two or more thereof.

Following application of the enteric coating, the pharmaceutical composition may have identifying information printed thereon using inks and procedures known in the art, such as offset gravure printing. Pharmaceutically acceptable inks that may be used with offset gravure printing include MARKEM™ 2200, 2202, 2212 and 2222, from Markem Corporation, Keene, New Hampshire U.S.A. These inks are typically shellac-based and contain pigments. Thinners may be added to any of these inks to increase or decrease the drying rate and/or modify the viscosity. Following application, these inks are normally air dried. Other pharmaceutically acceptable inks include those products sold as OPACODE™ and OPACODE™ WB, both of which contain pigments, titanium dioxide, and a solvent and are sold by Colorcon, West Point, Pennsylvania U.S.A. Many other printing inks are known to those skilled in the art, and any of these will be useful for the dosage forms of the invention.

Optionally, the enteric-coated dosage forms can be further coated with a thin film. Frequently, the film will be colored to facilitate product identification and for esthetic purposes; in this instance, any desired printing of information will be done after the film coating has been applied. Many suitable film coating products are commercially available, including those sold by Colorcon, West Point, Pennsylvania U.S.A using the OPADRY and OPAGLOS trademarks. These products from Colorcon are dry powders, containing a polymer, plasticizer, and pigment, that are mixed with water or a solvent such as alcohol, and sprayed onto tablets or other solid dosage forms. This film coating procedure, and alternative film coating products, are well known in the art.

In certain cases where the swellable coating is not further coated with an enteric coating, it still will be desired to imprint information on the dosage form. In many of such cases, or when an appearance enhancement such as a color is desired, the outer film coat generally will be useful.

The coated tablets, pellets, granules, or particulates may be encased in capsules for ease of administration. The encasement may be accomplished by any method known in the art, such as filling a pre-formed capsule. Such capsules may be comprised of gelatin or any other material known to those of skill in the art.

Without being limited to any theory, it is postulated that after an enteric coating dissolves in the intestine, or if there is no enteric coating, the swellable coating imbibes intestinal fluids and expands outwardly. Thus, initially, the swellable coating expands like a balloon being inflated and does not burst. As the coating swells, its permeability to water increases. It is hypothesized that the swellable coating contains microchannels, through which water enters by diffusion and reaches the core. The water causes the core to begin to fragment. Some of these fragments can puncture the swellable coating, leading to the ingress of more water. The additional water produces even more fragmentation of the core, which is thought to cause more fragments to puncture the swellable coating. This cycle is believed to continue until the pharmaceutical active is fully released or until the swellable coating is so weakened by the imbibition that the coating ruptures.

Further without being limited to any theory, it is believed that the release of the active may be modulated by several factors other than the presence of an enteric coating. One such factor is the selection of a hydrocolloid-forming substance in the swellable coating. Hydrocolloids vary in their swelling ability and hence their permeability to intestinal fluid. The permeability of the hydrocolloid is postulated to affect the hydration rate of the core and the resultant fragmentation of the core. Hydrocolloids also differ in tensile strength, which is thought to affect the percentage of core fragments that are able to puncture the swellable coating upon fragmentation. The number of fragments that are able to achieve egress directly affects the release of the pharmaceutical active. It is also believed that the number of openings created in the swellable coating further affects the release of the active by permitting more intestinal fluid into the core, producing more fragmentation. Tensile strength additionally affects whether and when a swellable coating ruptures due to the weakening caused by the imbibition of water, resulting in complete release of the active. Furthermore, some hydrocolloids erode upon swelling, which affects the ease with which core fragments are able to puncture the swellable coating.

Another factor can be the optional addition of an excipient to the swellable coating that modulates the release of the pharmaceutical active. Such agents can increase or decrease the permeability of the hydrocolloid to the intestinal fluid. This permeability affects the amount of intestinal fluid that contacts the core and leads to fragmentation. It is hypothesized that the fragments puncture the swellable coating upon fragmentation, thereby affecting the release of the pharmaceutical active. It is further believed that the openings created in the swellable coating provide conduits for the entry of additional intestinal fluid into the core, further accelerating fragmentation.

A third factor is the use of a disintegrant in the core. The employment of a disintegrant increases the rate of fragmentation of the core, which is thought to raise the frequency with which fragments create voids in the swellable coating. The sheer increase in fragments exiting through the swellable coating raises the rate of release of the pharmaceutical active. Additionally, the higher number of voids created in the swellable coating is believed to allow more water to enter the core, causing an even greater fragmentation of the active. Furthermore, the disintegrant may augment the force at which the core fragments impact the swellable coating, which may result in more fragments successfully creating voids in the swellable coating. These more forceful disintegrations further raise the rate of release of the pharmaceutical active by allowing a greater number of core fragments to pass through the swellable coating. Such disintegrants also produce additional openings for the intestinal fluid to hydrate and fragment the active, leading to additional release of the active

Although the rate of release of the pharmaceutical active can be modulated as set forth above, this invention does not have an objective of producing sustained release formulations whereby the pharmaceutical active is released at a controlled rate over an extended period of time, such as 12 or 24 hours. Rather, a feature of this invention is a delayed release of ingested pharmaceutical active until the dosage form has reached the intestinal tract, then facilitation of a rapid, essentially complete release of the pharmaceutical active for systemic absorption.

The following examples are provided to aid in understanding the invention, and are not intended, and should not be construed, to limit in any manner the invention as defined in the appended claims. In the examples, ingredients that are volatile during drying and therefore not present in the final product are not included in the tabular listings of ingredients; such ingredients, however, are mentioned as solvents, etc. in the preparation procedure discussions. Further, the weight added by printing information on a finished dosage form is insignificant and therefore is not included in the final cumulative weights. Percentages are expressed on a weight basis, unless the context clearly indicates otherwise.

### EXAMPLE 1

Tablets containing either 20 or 40 mg of pantoprazole were prepared using the following components and procedure:

| **Ingredients** | | **Quantity (mg) per 20 mg Tablet** | **Quantity (mg) per 40 mg Tablet** |
|---|---|---|---|
| **Core Tablet** | | | |
| | ***Dry Mixing*** | | |
| Pantoprazole sodium | | 22.55 | 45.1 |
| Mannitol (PEARLITOL SD-200) | | 110.95 | 221.9 |
| Crospovidone | | 8.25 | 16.5 |
| Sodium carbonate | | 3.75 | 7.5 |

| | ***Granulation*** | | |
|---|---|---|---|
| Sodium carbonate anhydrous | | 3.75 | 7.5 |
| Hydroxypropyl cellulose (KLUCEL LF) | | 4 | 8 |

| | ***Lubrication*** | | |
|---|---|---|---|
| Crospovidone | | 8.25 | 16.5 |
| Talc | | 1.5 | 3 |
| Calcium stearate | | 2 | 4 |
| | Total | 165 | 330 |

| **Swellable Coating** | | | |
|---|---|---|---|
| Zein F6000 | | 2.07 | 4.13 |
| Methacrylic acid copolymer (EUDRAGIT L 100-55) | | 0.41 | 0.82 |
| | Cum. Total | 167.48 | 334.95 |

| **Enteric Coating** | | | |
|---|---|---|---|
| Methacrylic acid copolymer (EUDRAGIT L 100-55) | | 9.25 | 18.49 |
| Triethyl citrate | | 0.93 | 1.85 |
| Titanium dioxide | | 1.83 | 3.65 |
| Talc | | 1.41 | 2.81 |
| | Cum. Total | 180.83 | 361.65 |

| **Film Coating** | | | |
|---|---|---|---|
| OPADRY Yellow OY-52945 | | 4.52 | 9.04 |
| | Cum. Total | 185.42 | 370.79 |

| **Printing** | | | |
|---|---|---|---|
| OPACODE Black S-1-8152 HV | | q.s. | q.s. |

Tablet cores were prepared by granulating a dry mix of pantoprazole sodium, mannitol, crospovidone and sodium carbonate with an aqueous solution of hydroxypropyl cellulose (KLUCEL LF from Hercules, Incorporated of Wilmington, Delaware U.S.A.) and sodium carbonate anhydrous. The granulates were dried using conventional drying techniques. The dried granules were then lubricated with crospovidone, talc and calcium stearate. The lubricated granules were compressed into cores. The cores were subcoated with a mixture of zein, EUDRAGIT L 100-55, water, and isopropyl alcohol, and dried. Enteric coating on top of the subcoat was performed using EUDRAGIT L 100-55 with isopropyl alcohol as the solvent and triethyl citrate as the plasticizer. Talc and titanium dioxide were used as the lubricant and the opaquent, respectively. After drying, the enteric coated tablet was film-coated using OPADRY Yellow OY-52945 and printed with OPACODE Black S-1-8152 HV.

### EXAMPLE 2

Tablets containing either 20 or 40 mg of pantoprazole were prepared using the following components and procedure.

| **Ingredients** | **Quantity (mg) per 20 mg Tablet** | **Quantity (mg) per 40 mg Tablet** |
|---|---|---|
| **Core Tablet** | | |
| ***Dry Mixing*** | | |
| Pantoprazole potassium | 22.55 | 45.1 |
| Mannitol (PEARLITOL SD-200) | 110.95 | 221.9 |
| Crospovidone | 8.25 | 16.5 |
| Sodium carbonate | 3.75 | 7.5 |

| ***Granulation*** | | |
|---|---|---|
| Sodium carbonate anhydrous | 3.75 | 7.5 |
| Hydroxypropyl cellulose (KLUCEL LF) | 4 | 8 |

| ***Lubrication*** | | |
|---|---|---|
| Crospovidone | 8.25 | 16.5 |
| Talc | 1.5 | 3 |
| Calcium stearate | 2 | 4 |
| Total | 165 | 330 |

| **Swellable Coating** | | |
|---|---|---|
| Zein F6000 | 2.07 | 4.13 |
| Methacrylic acid copolymer (EUDRAGIT L 100-55) | 0.41 | 0.82 |
| Cum. Total | 167.48 | 334.95 |

| **Enteric Coating** | | |
|---|---|---|
| Methacrylic acid copolymer (EUDRAGIT L 100-55) | 9.25 | 18.49 |
| Triethyl citrate | 0.93 | 1.85 |
| Titanium dioxide | 1.83 | 3.65 |
| Talc | 1.41 | 2.81 |
| Cum. Total | 180.83 | 361.65 |

| **Film Coating** | | |
|---|---|---|
| OPADRY Yellow OY-52945 | 4.52 | 9.04 |
| Cum. Total | 185.42 | 370.79 |

| **Printing** | | |
|---|---|---|
| OPACODE Black S-1-8152 HV | q.s. | q.s. |

Tablet cores were prepared by granulating a dry mix of pantoprazole sodium, mannitol, crospovidone and sodium carbonate with an aqueous solution of hydroxypropyl cellulose (KLUCEL LF) and sodium carbonate anhydrous. The granulates were dried using conventional drying techniques. The dried granules were lubricated with crospovidone, talc and calcium stearate. The lubricated granules were then compressed into cores. The cores were subcoated with a mixture of zein, EUDRAGIT L 100-55, water, and isopropyl alcohol. After drying, an enteric coating on top of the subcoat was performed using EUDRAGIT L 100-55 with isopropyl alcohol as the solvent and triethyl citrate as the plasticizer. Talc and titanium dioxide were used as the lubricant and the opaquent, respectively. Then, the dried enteric coated tablet was film-coated using OPADRY Yellow OY-52945 and printed upon with OPACODE Black S-1-8152 HV.

### EXAMPLE 3

Capsules containing 40 mg of omeprazole were prepared using the following components and procedure:

| **Ingredients** | **Quantity/Capsule (mg)** |
|---|---|
| **Core Pellets** | |
| Omeprazole | 40 |
| Mannitol | 236 |
| Crospovidone | 18 |
| Hydroxypropyl methylcellulose, 5 cps | 8 |
| Poloxamer 407 | 5 |
| Meglumine | 3 |
| Total | 310 |

| **Swellable Coating** | |
|---|---|
| Zein F 6000 | 6.2 |
| Cum. Total | 316.2 |

| **Enteric Coating** | |
|---|---|
| Hydroxypropyl methylcellulose phthalate (HP 55) | 63.24 |
| Triethyl citrate | 6.31 |
| Talc | 9.45 |
| Cum. Total | 395.25 |

Omeprazole core pellets were prepared by mixing omeprazole, mannitol, crospovidone, meglumine and polaxomer and granulating this mixture with hydroxypropyl methylcellulose as a binder. The granules thus obtained were subjected to extrusion and spheronization to produce spherical pellets. The pellets were then dried by conventional drying techniques. The pellets were coated with a swellable coating containing zein and sodium lauryl sulfate dissolved in a mixture of isopropyl alcohol and water, then dried. The enteric coat was prepared by dissolving hydroxypropyl methylcellulose phthalate and triethyl citrate in a mixture of isopropyl alcohol and acetone and dispersing talc in this solution, which was then layered upon the intermediate coating.

The coated pellets were measured into a gelatin capsule.

### EXAMPLE 4

Tablets containing 40 mg of omeprazole were prepared using the following ingredients and procedure:

| **Ingredients** | **Quantity/Tablet (mg)** |
|---|---|
| **Core Tablet** | |
| Omeprazole | 40 |
| Mannitol (PEARLITOL SD-200) | 231.3 |
| Crospovidone | 6 |
| Meglumine | 3 |
| Poloxamer 407 | 5 |
| Hydroxypropyl methylcellulose, 5 mPa·s | 8 |
| Magnesium stearate | 3.8 |
| Talc | 3 |
| Total | 300 |

| **Swellable Coating** | |
|---|---|
| Zein F 6000 | 2.73 |
| Sodium lauryl sulfate | 0.27 |
| Cum. Total | 303 |

| **Enteric Coating** | |
|---|---|
| Hydroxypropyl methylcellulose phthalate (HP 55) | 24 |
| Triethyl citrate | 2.4 |
| Talc | 3.6 |
| Cum. Total | 333 |

Omeprazole core tablets were prepared by mixing omeprazole, mannitol, crospovidone, meglumine and poloxmer and granulating the mixture with hydroxypropyl methylcellulose as a binder. The granules were dried in fluid bed drier and the dried granules were compressed into tablets or minitablets. These core tablets or minitablets were coated with intermediate coating solution containing zein and sodium lauryl sulfate dissolved in a mixture of isopropyl alcohol and water, then dried. The enteric coat was prepared by dissolving hydroxypropyl methylcellulose phthalate and triethyl citrate in a mixture of isopropyl alcohol and acetone and dispersing talc in this solution, which was then layered upon intermediate coating.

### EXAMPLE 5

Tablets containing 40 mg of pantoprazole were prepared using the following components and procedure:

| **Ingredients** | **Quantity/Tablet (mg)** |
|---|---|
| **Core Tablet** | |
| Pantoprazole sodium sesquihydrate | 45 |
| Mannitol (PEARLITOL SD-200) | 143.18 |
| Mannitol (PEARLITOL DC-400) | 47.72 |
| Crospovidone | 16.5 |
| PLASDONE S-630 | 30 |
| Sodium lauryl sulfate | 2.5 |
| Meglumine | 3 |
| Calcium stearate | 6 |
| Talc | 6 |
| Total | 300 |

| **Swellable Coating** | |
|---|---|
| Zein | 4.5 |
| Cum. Total | 304.5 |

| **Enteric Coating** | |
|---|---|
| Methacrylic acid copolymer (EUDRAGIT L100-55) | 16.81 |
| Triethyl citrate | 1.68 |
| Titanium dioxide | 3.39 |
| Talc | 2.51 |
| Cum. Total | 328.89 |

Core tablets were prepared by blending pantoprazole sodium sesquihydrate with mannitol, crospovidone, PLASDONE S630, talc, and magnesium stearate, and direct compressing into tablets. These core tablets were coated with a swellable coating solution containing zein and sodium lauryl sulfate dissolved in a mixture of isopropyl alcohol and water, then dried. The enteric coat was prepared by dissolving hydroxypropyl methylcellulose phthalate and triethyl citrate in a mixture of isopropyl alcohol and acetone and dispersing talc in this solution, which was then layered upon intermediate coating.

### EXAMPLE 6

Capsules containing esomeprazole were prepared using the following components and procedure:

| **Ingredients** | **Quantity (g)** |
|---|---|
| **Pellets** | |
| Esomeprazole magnesium trihydrate | 178 |
| Mannitol | 938 |
| Crospovidone | 72 |
| Sodium lauryl sulfate | 20 |
| Copovidone | 32 |
| Total | 1240 |

| **Swellable Coating** | |
|---|---|
| Zein | 16.2 |
| Sodium lauryl sulfate | 1.62 |
| Cum. Total | 1257.82 |

| **Enteric Coating** | |
|---|---|
| Methacrylic acid copolymer, Type C | 110 |
| Triethyl citrate | 11 |
| Titanium dioxide | 15.29 |
| Talc | 16.5 |
| Cum. Total | 1410.61 |

The core was prepared by mixing esomeprazole magnesium trihydrate, mannitol, crospovidone and sodium lauryl sulfate and granulating this mixture with an aqueous solution of copovidone. The granules were then subjected to extrusion and spheronization to obtain spherical pellets. The pellets were dried by conventional drying techniques. The dried pellets were coated with intermediate coating solution containing zein and sodium lauryl sulfate dissolved in a mixture of isopropyl alcohol and water, then dried. The enteric coat was prepared by dissolving Methacrylic acid copolymer, Type C and triethyl citrate in isopropyl alcohol, and dispersing talc and titanium dioxide in this solution.

Coated pellets are filled into gelatin capsules, giving 4000 capsules that each contain 40 mg of esomeprazole.

### EXAMPLE 7

Esomeprazole tablets were prepared, using the following ingredients and procedure.

| **Ingredients** | **Quantity (mg/tablet)** |
|---|---|
| **Core Tablet** | |
| Esomeprazole magnesium trihydrate | 44.5 |
| Magnesium oxide | 20 |
| PLASDONE S-630 | 17.5 |
| Crospovidone | 10 |
| Mannitol (PEARLITOL SD 200) | 227 |
| Colloidal silicon dioxide | 3.5 |
| Sodium stearyl fumarate | 17.5 |
| Total | 340 |

| **Swellable Coating** | |
|---|---|
| Zein F6000 | 6.8 |
| Cum. Total | 346.8 |

| **Enteric Coating** | |
|---|---|
| EUDRAGIT L100-55 | 19.1 |
| Triethyl citrate | 1.9 |
| Titanium dioxide | 3.8 |
| Talc | 2.9 |
| Cum. Total | 374.5 |

Esomeprazole magnesium trihydrate, magnesium oxide, copovidone, crospovidone, mannitol, and silicon dioxide were blended, then sodium stearyl fumarate was added with further blending. This mixture was compressed into core tablets. The tablets were coated with an aqueous alcohol solution of zein, then dried. Finally, the enteric coating ingredients were dispersed in water and coated onto the zein-coated tablets, followed by a final drying.

### EXAMPLE 8

Tablets containing rabeprazole sodium were prepared using the following components and procedure:

| **Ingredients** | **Quantity/Tablet (mg)** |
|---|---|
| **Core Tablet** | |
| Rabeprazole sodium | 20 |
| Mannitol (PEARLITOL SD 200) | 97.2 |
| Mannitol (PEARLITOL DC 400) | 28 |
| Meglumine | 5.1 |
| Crospovidone | 3.4 |
| PLASDONE S-630 | 10.5 |
| Talc | 3.4 |
| Magnesium stearate | 2.4 |
| Total | 170 |

| **Swellable Coating** | |
|---|---|
| Zein F6000 | 4.25 |
| Triethyl citrate | 0.2 |
| Cum. Total | 174.45 |

| **Enteric Coating** | |
|---|---|
| Methacrylic acid copolymer (EUDRAGIT L 100-55) | 12.26 |
| Triethyl citrate | 1.224 |
| Talc | 0.68 |
| Cum. Total | 188.614 |

Rabeprazole sodium, crospovidone, PLASDONE S630 and mannitol (PEARLITOL SD 200) were mixed with mannitol (PEARLITOL DC 400) for 20 minutes. Talc and magnesium stearate were then added to the mixture and mixed for 5 minutes. This lubricated blend was then compressed into tablets. The core tablets were subcoated with a water-alcohol solution of zein (weight increase 2.5±0.5%) and dried. The subcoated tablets were coated with enteric coating solution (weight increase 8-9%).

### EXAMPLE 9

Rabeprazole sodium tablets were prepared using the following components and procedure:

| **Ingredients** | **Quantity/Tablet (mg)** |
|---|---|
| **Core Tablet** | |
| Rabeprazole sodium | 20 |
| Mannitol (PEARLITOL SD-200) | 97.01 |
| Low substituted hydroxypropyl cellulose, LH21 ("L-HPC") | 14.4 |
| Magnesium oxide | 40 |
| Sodium lauryl sulfate | 1.8 |
| Hydroxypropyl methylcellulose, 5 mPa·s | 3 |
| Talc | 1.54 |
| Magnesium stearate | 2.25 |
| Total | 180 |

| **Swellable Coating** | |
|---|---|
| Zein 6000 | 4.9 |
| Triethyl citrate | 0.49 |
| Cum. Total | 185.39 |

| **Enteric Coating** | |
|---|---|
| EUDRAGIT L100-55 | 14.46 |
| Triethyl citrate | 1.44 |
| Talc | 0.79 |
| Cum. Total | 202.08 |

| **Film Coating** | |
|---|---|
| OPADRY Yellow OY-52945 | 5.05 |
| Cum. Total | 207.13 |

| **Printing** | |
|---|---|
| OPACODE Black | q.s. |

Magnesium oxide was sifted through a 60 mesh sieve. Rabeprazole sodium, L-HPC, mannitol and the sifted magnesium oxide were sifted through a 40 mesh sieve. The materials were then mixed for 30 minutes in a Rapid mixer granulator. Sodium lauryl sulfate (SLS) was dissolved in purified water and hydroxypropylmethylcellulose (HPMC) was dissolved in warm purified water. The rabeprazole sodium mixture was mixed with the SLS and HPMC solutions. The wet mass was dried in a fluid bed drier and the dried granules were sifted through a 20 mesh sieve. The sifted granules were blended with L-HPC in a double cone blender for 5 minutes. Magnesium stearate (sifted through a 60 mesh sieve) was added to the blend and mixed for 5 minutes. The lubricated blend was then compressed into core tablets. The core tablets were coated with a water-alcohol zein coating solution (weight increase 2.5 ± 0.5%) and dried. The coated tablets were further coated with enteric coating solution (weight increase 8.0 ± 1.0%). The enteric coated tablets were additionally coated with OPADRY solution until the weight increase was 2.0 ± 0.5%. Then, the film coated tablets were imprinted with OPACODE black ink.

### EXAMPLE 10

Pantoprazole sodium tablets, prepared according to Example 5, were tested according to Method 724 "Drug Release" of *The United States Pharmacopeia 24,* United States Pharmacopeial Convention, Inc., Rockville, Maryland U.S.A., pp. 1944-1947, 2000, using Method B and Apparatus 1 (described in Method 711 "Dissolution," on page 1942). A tablet was first immersed in 0.1 N hydrochloric acid, with stirring, for two hours at 37°C. The tablet was then immersed in the pH 6.8 phosphate buffer, with stirring, and samples of the buffer solution were taken at intervals for analysis to determine the amount of drug released from the tablet.

Following are the data obtained from testing six tablets. The amount of drug released into the acid is not shown, but was small. In general, release of up to 10% of the drug into the acid is considered acceptable for enteric coated dosage forms. For purposes of this invention, a pharmaceutical active is considered to be substantially retained within the dosage form if less than about ten percent by weight is released into 0.1 N hydrochloric acid, under the conditions of the USP test.

| **Time (min.)** | **Percent Drug Released** | | | | | | |
|---|---|---|---|---|---|---|---|
| | **Tablet 1** | **Tablet 2** | **Tablet 3** | **Tablet 4** | **Tablet 5** | **Tablet 6** | **Mean** |
| 0 | 0 | 0 | 0 | 0 | 0 | 0 | 0 |
| 15 | 29 | 18 | 22 | 21 | 18 | 17 | 21 |
| 30 | 61 | 62 | 65 | 57 | 55 | 58 | 60 |
| 45 | 82 | 86 | 84 | 84 | 81 | 79 | 83 |
| 60 | 92 | 94 | 91 | 92 | 89 | 88 | 91 |

These results show that the drug was substantially completely released within sixty minutes at pH 6.8.

### EXAMPLE 11

As in Example 10, tablets of rabeprazole sodium prepared according to Example 9 were tested by USP Drug Release Method 724. However, the alkaline solution for the second part of the test was a phosphate buffer adjusted to pH 8.0 and also containing 0.5 weight percent of sodium lauryl sulfate. Results were obtained, as follows.

| **Time (min.)** | **Percent Drug Released** | | | | | | |
|---|---|---|---|---|---|---|---|
| | **Tablet 1** | **Tablet 2** | **Tablet 3** | **Tablet 4** | **Tablet 5** | **Tablet 6** | **Mean** |
| 0 | 0 | 0 | 0 | 0 | 0 | 0 | 0 |
| 10 | 0 | 0 | 0 | 0 | 0 | 0 | 0 |
| 20 | 0 | 0 | 0 | 0 | 0 | 0 | 0 |
| 30 | 18 | 37 | 12 | 43 | 0 | 28 | 23 |
| 45 | 82 | 94 | 74 | 94 | 93 | 92 | 88 |
| 60 | 91 | 91 | 92 | 90 | 92 | 92 | 91 |

These results show that the drug was substantially completely released within sixty minutes at pH 6.8.

### EXAMPLE 12

Esomeprazole tablets were prepared using the following ingredients and the procedure described below.

| **Ingredients** | **Quantity/Tablet (mg)** |
|---|---|
| **Core Tablet** | |
| Esomeprazole magnesium trihydrate | 44.5 |
| Magnesium oxide | 20 |
| PLASDONE S-630 | 17.5 |
| Mannitol (PEARLITOL SD 200) | 237 |
| Colloidal silicon dioxide | 3.5 |
| Sodium stearyl fumarate | 17.5 |
| Total | 340 |

| **Swellable Coating** | |
|---|---|
| Zein F6000 | 6.8 |
| Cum. total | 346.8 |

Esomeprazole magnesium trihydrate, magnesium oxide, PLASDONE S-630, silicon dioxide, and mannitol were sieved and blended, then sodium stearyl fumarate was added and the mixture blended, and finally tablets were formed by direct compression of the mixture. Zein was dissolved in aqueous alcohol and coated onto the tablets. The coated tablets were then dried.

Additional tablets were similarly prepared, further including either 7 mg or 10 mg of the disintegrant ingredient crospovidone in the core composition, with corresponding decreases in the amount of mannitol to maintain constant tablet weights. The tablets were tested for dissolution characteristics at pH 6.8, using the procedure of Example 10 (except that the acid contact step was omitted) and giving the following results.

| **Time (min.)** | **Percent Drug Released** | | |
|---|---|---|---|
| | **No Disintegrant** | **7 mg Disintegrant** | **10 mg Disintegrant** |
| 15 | 0 | 0 | 61 |
| 30 | 0 | 1 | 80 |
| 45 | 0 | 3 | 86 |
| 60 | 0 | 6 | 89 |
| 90 | 0 | - | 88 |
| 120 | 1 | - | - |

For this particular formulation, 10 mg of disintegrant produced the desired rapid release of drug at pH 6.8. However, other formulations could exhibit the desired drug release with different disintegrant concentrations, depending on the identity of the various formulation components, the physical methods used to prepare cores (such as compression pressure for tablets), and the presence of additional coatings. Therefore, each proposed formulation should be tested using varying amounts of the selected disintegrant components, to identify the exact formulation that gives desired drug release characteristics.

### EXAMPLE 13

Capsules containing 40 mg of esomeprazole were prepared, using the following:

| **Ingredients** | **Quantity/Capsule (mg)** |
|---|---|
| **Core** | |
| Esomeprazole magnesium trihydrate | 44.5 |
| Mannitol | 229.5 |
| Crospovidone | 18 |
| Sodium lauryl sulfate | 10 |
| Copovidone | 8 |

The core ingredients were blended and granulated with water, then the mixture was extruded and spheronized to form pellets. After drying, the pellets were provided with a swellable subcoating to a weight gain of 3.3-3.5 percent, using Zein F6000 and a commercial product containing a methacrylic acid copolymer (EUDRAGIT L100-55) and triethyl citrate, in aqueous isopropanol, and drying to remove the solvents. The subcoated pellets were then given an enteric coating with a mixture of talc and a commercial product containing a methacrylic acid copolymer (EUDRAGIT L100-55) and triethyl citrate, in isopropanol, and the solvent was removed by drying. The enteric coated pellets were filled into a hard gelatin capsule.

### EXAMPLE 14

Capsules prepared according to Example 13 were tested for dissolution in different media, simulating physiologic conditions while fasting and while food is present. Commercially available capsules of NEXIUM™ esomeprazole magnesium trihydrate capsules from AstraZeneca LP of Wilmington, Delaware U.S.A. were also subjected to the dissolution testing; these capsules are described in their prescribing literature as having delayed-release properties and containing 40 mg of esomeprazole as esomeprazole magnesium trihydrate in the form of enteric-coated pellets, having the following inactive ingredients: glyceryl monostearate 40-50, hydroxypropyl cellulose, hypromellose, magnesium stearate, methacrylic acid copolymer type C, polysorbate 80, sugar spheres, talc, and triethyl citrate.

The dissolution test was conducted according to *United States Pharmacopeia 24,* Test 711, using Apparatus 1 and rotating the basket at 100 rpm. The various dissolution media were as follows:

| **pH 2.1 (Stomach - Fasting)** | |
|---|---|
| **Ingredient** | **Quantity** |
| Sodium chloride | 2 grams |
| Hydrochloric acid, 35 wt. percent | 0.85 mL |
| Sodium lauryl sulfate | 2.5 grams |
| Water | q.s. for 1000 mL |

| **pH 5 (Intestine - Fed)** | |
|---|---|
| **Ingredient** | **Quantity** |
| Sodium taurocholate | q.s. for 15 mM |
| Lecithin | q.s. for 3.75 mM |
| Sodium hydroxide | 4.04 grams |
| Glacial acetic acid | 8.65 grams |
| Sodium chloride | 11.874 grams |
| Water | q.s. for 1000 mL |

| **pH 6.8 (Intestine - Fasting)** | |
|---|---|
| **Ingredient** | **Quantity** |
| Potassium hydrogen phosphate | 29 mM |
| Sodium taurocholate | 5 mM |
| Lecithin | 1.5 mM |
| Potassium chloride | 0.22 mM |
| Sodium hydroxide | q.s. for pH 6.8 |
| Water | q.s. |

The results of this testing were as follows:

| **Condition** | **NEXIUM** | **Example 13** |
|---|---|---|
| pH 2.1 | Pellets disintegrated in 30-40 minutes; yellow solution | Pellets were intact after one hour; colorless solution |
| pH 5 | 34-36 percent of the contained esomeprazole degraded in 30-40 minutes | 7-10 percent of the contained esomeprazole degraded in one hour |
| pH 6.8 | > 90 percent of the contained esomeprazole dissolved; no degradation | > 90 percent of the contained esomeprazole dissolved; no degradation |

The yellow solution observed from testing NEXIUM capsules at pH 2.1 is considered to be a result of a complete acid degradation of the esomeprazole.

### EXAMPLE 15

An *in vivo* bioavailability test under fed and fasting conditions was conducted to compare the capsules from Example 13 with NEXIUM capsules. Fourteen subjects were evaluated in a two-way crossover study, giving the following results:

| **Test** | **AUC**_{**0-t**} | **AUC**_{**0-∞**} | **C**_{**max**} | **T**_{**max**} |
|---|---|---|---|---|
| Example 13, Fed | 4019 ng•hr/mL | 4042 ng•hr/mL | 1080 ng/mL | 6.25 Hr. |
| Example 13, Fasted | 4069 ng•hr/mL | 4217 ng•hr/mL | -- | -- |
| Example 13 Fed/Fasted | 99.43 % | 96.49 % | -- | -- |
| NEXIUM, Fed | 2160 ng•hr/mL | 2261 ng•hr/mL | 635 ng/mL | 6.25 Hr. |
| NEXIUM, Fasted | 4328 ng•hr/mL | 4400 ng•hr/mL | -- | -- |
| NEXIUM Fed/Fasted | 49.00 % | 50.92 % | -- | -- |

where Cₘₐₓ is the mean of maximum plasma concentrations after dosing, Tₘₐₓ is the elapsed time after dosing for obtaining the Cₘₐₓ value, AUC₀₋ₜ is the integrated area under the curved obtained by plotting plasma concentration versus time since dosing, beginning at zero time and ending at the last time of a measurable plasma concentration of the drug, and AUC_{0-∞} is the integrated area under the curved obtained by plotting plasma concentration versus time since dosing, beginning at zero time and ending at an assumed complete elimination of drug from the system.

### EXAMPLE 16

Esomeprazole tablets were prepared, containing the following:

| **Ingredient** | **Mg/Tablet** |
|---|---|
| **Core** | |
| Esomeprazole (40 mg) + mannitol (37 mg) + meglumine (3 mg) mixture | 80 |
| Mannitol | 158 |
| Crospovidone | 22 |
| Magnesium oxide | 20 |
| Glycine | 17 |
| Sodium lauryl sulfate | 3.5 |
| Colloidal silicon dioxide | 1 |
| Copovidone | 25 |
| Talc | 3 |
| Sodium stearyl fumarate | 10 |

| **Swellable coating** | |
|---|---|
| Zein | 6.8 |

| **Enteric coat** | |
|---|---|
| Methacrylic acid copolymer (EUDRAGIT L100-55) | 20.8 |
| Triethyl citrate | 2.08 |
| Titanium dioxide | 0.52 |
| Talc | 0.39 |

The core components were mixed and compressed into tablets. Zein was dissolved in a mixture of isopropanol and water, coated onto the tablets, and the coated tablets were dired to remove solvents. The enteric coating components, in an isopropanol vehicle, were coated onto the zein-coated tablets, and the solvent was removed by drying.

The tablets were tested for their dissolution characteristics, under different pH conditions. Using the methodology of preceding Example 14, nineteen subjects were evaluated

### EXAMPLE 17

Tablets prepared in Example 16 were used for an *in vivo* bioavailability test under fed conditions. Nineteen subjects were evaluated in a two-way crossover study, similarly to Example 15, giving the following results:

| **Test** | **AUC**_{**0-t**} | **AUC**_{**0-∞**} | **Cmax** | **Tmax** |
|---|---|---|---|---|
| Example 16 | 3950 µg·hr/mL | 4082 µg·hr/mL | 1292 µg/mL | 5 Hr. |
| NEXIUM | 2327 µg·hr/mL | 2453 µg·hr/mL | 537 µg/mL | 6 Hr. |
| Example 16/NEXIUM | 1.7 | 1.7 | 2.4 | -- |

These results show the enhanced bioavailability parameters obtained with the Example 16 dosage form.

In the pharmaceutical dosage forms according to the invention, the core may also comprise between 91.5% and 97.5% of the weight of the dosage form, between 91.5% and 95% of the weight of the dosage form, between 93% and 97.5% of the weight of the dosage form, or between 95% and 97.5% of the weight of the dosage form. The enteric coating may comprise between 4% and 7% of the weight of the dosage form, between 5% and 7% of the weight of the dosage form, between 6% and 7% of the weight of the dosage form, between 3% and 6% of the weight of the dosage form, or between 4% and 6% of the weight of the dosage form. The swellable coating may comprise between 1.5% and 3% of the weight of the dosage form, between 1.5% and 2.5% of the weight of the dosage form, between 1.5% and 2% of the weight of the dosage form, between 2% and 3% of the weight of the dosage form, or between 2.5% and 3% percent of the weight of the dosage form.

There have been disclosed hereinbefore the methods defined by the following numbered paragraphs.
1. A method of treatment, comprising administering a pharmaceutical dosage form comprising:
   a. a solid core comprising a pharmaceutical active and a disintegrant; and
   b. a swellable coating surrounding the core; and providing equivalent bioavailability of the pharmaceutical active whether the dosage form is administered in a fasted or a fed state.
2. A method of treatment comprising administering a pharmaceutical dosage form comprising:
   a. a solid core comprising an acid-sensitive pharmaceutical active and a disintegrant;
   b. a swellable coating comprising a hydrocolloid-forming component, surrounding the core; and
   c. an enteric coating surrounding the swellable coating; and providing equivalent bioavailability of the pharmaceutical active whether the dosage form is administered in a fasted or a fed state.
3. A method of treatment comprising administering a pharmaceutical dosage form comprising:
   a. a solid core comprising a benzimidazole and a disintegrant;
   b. a swellable coating comprising one or more hydrocolloid-formers selected from zein, crospovidone, and a hydroxypropyl cellulose, surrounding the core; and
   c. an enteric coating comprising a copolymer of methacrylic acid and ethyl acrylate, surrounding the swellable coating;
   and providing equivalent bioavailability of the benzimidazole whether the dosage form is administered in a fasted or a fed state.
4. A method of treatment for minimizing inter-patient bioavailability differences, comprising administering a pharmaceutical dosage form comprising:
   a. a solid core comprising a pharmaceutical active and a disintegrant; and
   b. a swellable coating surrounding the core;
   wherein:
   1. the dosage form remains substantially intact during stomach transit;
   2. aqueous fluids penetrate areas of the dosage form, causing hydrocolloid formation in the swellable coating;
   3. aqueous fluids pass through the hydrocolloid to hydrate the core; and
   4. the hydrated core becomes fragmented, releasing the pharmaceutical active from the dosage form.

## Claims

1. A pharmaceutical dosage form comprising:
a solid core comprising a pharmaceutical active and a disintegrant;
a swellable coating surrounding the core; and
an enteric coating surrounding the swellable coating;
wherein the enteric coating comprises between 3% and 7% of the weight of the dosage form; and wherein the dosage form provides equivalent bioavailability whether administered to a fasted or fed subject.

2. A pharmaceutical dosage form according to claim 1 wherein the core comprises at least 91.5 percent of the weight of the dosage form.

3. A pharmaceutical dosage form according to claim 1 or 2 wherein the swellable coating comprises at least 1.5 percent of the weight of the dosage form.

4. A pharmaceutical dosage form according any preceding claim, wherein the core is a tablet.

5. A pharmaceutical dosage form according to any preceding claim, comprising multiple coated cores contained in a capsule.

6. A pharmaceutical dosage form according to any preceding claim, wherein the pharmaceutical active is unstable in the presence of acid.

7. A pharmaceutical dosage form according to any preceding claim, wherein the pharmaceutical active comprises a benzimidazole.

8. A pharmaceutical dosage form according to any preceding claim, wherein the benzimidazole is one or more of omeprazole, esomeprazole, lansoprazole, rabeprazole and pantoprazole.

9. A pharmaceutical dosage form according to any preceding claim, wherein the disintegrant comprises one or more members selected from the group consisting of: starches; polyvinyl pyrrolidones; formaldehyde-casein compounds; resins; defatted soybean extracts; alginic acid; agar-agar; calcium carbonate, calcium phosphate; sodium carbonate; and acrylic polymers.

10. A pharmaceutical dosage form according to any preceding claim, wherein the swellable coating comprises one or more hydrocolloid-forming members selected from the group consisting of: prolamines, vinylpyrrolidone polymers, cellulose derivatives; starches, carboxyvinyl polymers; alginates; pectins; agar; and gums.

11. A pharmaceutical dosage form according to any preceding claim, wherein the swellable coating comprises zein, hydroxypropylmethyl cellulose, hydroxypropylcellulose or crospovidone.

12. A pharmaceutical dosage form according to any preceding claim, wherein the swellable coating comprises an excipient that modulates release of pharmaceutical active from the core upon hydration.

13. A pharmaceutical dosage form of claim 12, wherein the excipient comprises one or more members selected from the group consisting of:
plasticizers; water soluble surfactants; and enteric coating materials.

14. A pharmaceutical dosage form according to any preceding claim, wherein the enteric coating comprises a component that is cellulose-based, methacrylate-based, polyvinyl acetate phthalate-based, or shellac-based.

15. A pharmaceutical dosage form according to any preceding claim, wherein the enteric coating comprises a copolymer of methacrylic acid and ethyl acrylate.

16. A pharmaceutical dosage form according to any preceding claim, wherein the pharmaceutical active is substantially retained in the dosage form while the dosage form is present in the stomach, but is rapidly released after the dosage form enters a digestive system environment having a pH value at least about 5.

17. The use, in the manufacture of a medicament for treatment of a medical condition, or for minimizing inter patient bioavailability differences, of a pharmaceutical active, wherein the medicament is orally administered as a dosage form comprising:
a solid core comprising the pharmaceutical active and a disintegrant;
a swellable coating surrounding the core; and
an enteric coating surrounding the swellable coating;
wherein the enteric coating comprises between 3 and 7% of the weight of the dosage form; and wherein the medicament is administered such that the dosage form provides equivalent bioavailability whether administered to a fasted or fed subject.

18. The use according to claim 17, wherein the dosage form is orally administered and wherein
a. the dosage form remains substantially intact during stomach transit;
b. aqueous fluids penetrate areas of the dosage form, causing hydrocolloid formation in the swellable coating;
c. aqueous fluids pass through the hydrocolloid to hydrate the core; and
d. the hydrated core becomes fragmented, releasing the pharmaceutical active from the dosage form.

19. The use according to any of claims 17 or 18, wherein at least about 80 percent of the pharmaceutical active is released within about one hour after the dosage form is contacted with an aqueous fluid having a pH about 6.8.
